# EUROPEAN PATENT SPECIFICATION

(11) **EP 0 955 024 B1**
(45) Date of publication and mention of the grant of the patent: **07.09.2005**
(21) Application number: 99201409.2
(22) Date of filing: 04.05.1999
(51) Int. Cl.: A61F 6/08, A63B 23/20, A61B 5/107, A61H 21/00

(54) **Intravaginal set and method of treatment of prolapse of the urogenital organs**
Intravaginaler Elementensatz und Verfahren zur Behandlung eines Prolapses der urogenitalen Organen
Jeu d'éléments intravaginaux et procédé de traitement d'un prolapsus des organes urogénitaux

(30) Priority: 04.05.1998 PL 32614198
(43) Date of publication of application: 10.11.1999
(60) Divisional application: 05010738.2
(73) Proprietor: Adamed SP. Z O.O., 05-152 Czosnow k/Warszawy (PL)
(72) Inventor: Adamkiewicz, Marian, 01-561 Warsaw (PL)
(74) Representative: HOFFMANN EITLE

(56) References cited:
- EP-A- 0 392 854
- DE-A- 2 121 790
- NL-C- 1 001 596
- US-A- 3 938 504
- US-A- 4 240 412

## Description

The present invention relates to an intravaginal set, used in the case of prolapse of the urogenital organs and urinary stress incontinence, or in the period of intervals, when a therapeutic intravaginal insert for treatment of static disorders of the urogenital organs and urinary stress incontinence is not currently inserted.

Aging and past parturitions result in weakening and elongation of the perineum muscles leading to the urogenital organs prolapse and other anatomical disorders. Once extended, muscles become weaker and weaker which subsequently results in progress of prolapse up to transvaginal eversion of the uterus. As the vaginal canal is the "locus minoris resistentiae" in the pelvic floor, walls of the vagina may become the ring of hernia (cystocele, uretrocele and rectocele).

In the course of progressive prolapse of the urogenital organs, discomfort in lower abdominal part intensifies, from "heaviness" to pain, and urinary stress incontinence becomes apparent.

Prolapse of the urogenital organs causes decrease in distance between the uterine cervix and the vaginal inlet, descent of the anterior wall of the vagina along with the urinary bladder and urethra (the cysto-urethral angle becomes more obtuse), and urethra dislocates outside an operation range of the intra-abdominal pressure.

This results in impaired blood outflow from the urogenital organs due to venous constriction (low-pressure blood vessels) .
In less advanced cases of the urogenital prolapse, special therapeutic exercises are recommended to strengthen the pelvic floor muscles.

The physical exercises may increase the efficiency of the cross-striated muscles only, i.e. muscles dependent an our own will, whereas the urogenital organs equilibrium is also maintained by independent-of-our-will smooth muscles, controlled by the autonomous nervous system. Smooth muscles build the urinary bladder, urethra, internal sphincter muscle of urethra, and muscles in uterine ligaments fixing uterus in its normal position.

A significant progress in the treatment of static disorders of the urogenital organs has been made by development of a therapeutic insert (the subject of Polish patent specification no RP 138406), consisting of a hollow ball with string attached freely outside the ball, and the smaller metal ball, placed freely inside. This device generates mechanical impulses stimulating contractions of surrounding muscles, both smooth, and cross-striated. A metal ball placed freely in the intravaginal therapeutic insert generates the mechanical impulses by hitting its cap as a result of the centre of gravity translocation while walking. The mechanical impulse stimulates muscular contraction. Regular muscular exercise results in muscles hypertrophy and an increase in muscular force. The therapeutic insert may be applied twice a day, over the period of about 30 minutes.

Prolonged application longer than 30 minutes leads to muscular overstrain and abdominal pain. An application of the therapeutic insert every day over the period of 3 months leads to satisfactory therapeutic results in the treatment of minor prolapse of the urogenital organs (grade I), while in mere advanced cases (grades II, III, IV) no improvement was observed.

The most probable explanation is such, that in more advanced urogenital prolapse, the uterus lowers during intervals between applications of therapy, which results in unfavourable effects (isometric contraction, passive congestion).

When conservative therapy is ineffective, the reconstructive surgery is performed to restore the normal position of the urogenital organs.

Different strategies of therapeutic management are used, depending an symptoms, age and progress of the illness. From among around 200 modifications applied to correct the position of the urogenital organs, the basic management consists in shortening of muscles and ligaments. Such therapeutic management ameliorates the position, but it does not restore the normal muscular function and thus the lasting and complete recovery.

In the case of contraindications for surgery, the device prostheses may be applied to ensure a normal position of the urogenital organs.

One of them is well-known intravaginal disc for support of lowered or prolapsed uterus. This disc is applied in such manner, that its ring surrounds the uterine cervix, thus preventing the uterus prolapse by extension of vaginal wall in the area of the posterior vaginal fornix. A disadvantage of this design consists in difficulties in application and removal, what makes its use impossible, when the therapeutic insert is not currently used. As the disc is placed in the upper part of the vagina, it does not transmit the contraction of the levator ani muscle, thus the uterus is not elevated.

The respective British patent application discloses a plastic planar arc, the wider arm of which rests an the pubic symphysis, while the second, narrow arm presses the urethra against the urinary bladder. The maximal time for intravaginal application is 2 hours which limits the usefulness of this device in unlimited time. Mode of action consists in local compression of the urethra, what may lead to inflammation and decubitus ulcers.

There is also a well-known disposable vaginal pack (made in Germany), which is placed in the vagina over period up to 8 hours. Its mode of action consists in mild compression of urethra and adjustment to the vagina after soaking with water before intravaginal application. This vaginal pack, however, does not meet the corrective functions, and may cause inflammatory state in the case of prolonged intravaginal presence. Soaking with vaginal secretion may result in distension of the vagina leading to progressive prolapse of the uterus and urinary bladder.

Applicant's corresponding US Patent No. 6,530,879 includes a recitation of prior art which fulfils the duty of candour on the part of Applicant towards the United States Patent and Trademark Office. The list of prior publications include two papers by Morton et al which report on Applicant's own KOLPEXIN T (trademark) device that corresponds to the "therapeutic insert" described in the present specification. See US-A-4574791 for disclosure of a vaginal insert on a cable for training the pubo coccygeus muscle in a rhythmic contraction and relaxation that reciprocates the insert in the vaginal cavity.

The object of the present invention is to design an intravaginal set for a method of treatment to obtain permanent optimal position of the uterus and urinary bladder. An intravaginal set according to the invention is useful in the case of prolapse of urogenital organs and urinary stress incontinence, or in the period of intervals in women when an intravaginal therapeutic insert for treatment of static disorders of the urogenital organs and urinary stress incontinence is not currently inserted, which therapeutic insert consists of hollow plastic ball with string attached and freely moving outside the ball, and with smaller ball, placed freely inside said hollow plastic ball, which smaller ball has a weight adequately adjusted to generate mechanical impulses stimulating alternate contractions of the smooth muscles, said intravaginal set comprising a sub-set of intravaginal corrective inserts and an intravaginal measuring sub-set for determining the size of the insert wherein the sub-set of intravaginal corrective inserts comprises at least two balls with step increase in diameter, ranging between the minimal and maximal woman vaginal diameter, each ball being preferably hollow, each ball has the loosely hanging string and each ball is made preferably of medical material, such as polycarbonate or methyl methacrylate, while the intravaginal measuring sub-set comprises at least two balls made of metal or plastic with graduated diameters corresponding to graduated diameters of the balls of sub-set of intravaginal corrective inserts, which measuring balls instead of the loosely hanging string, have a rigidly mounted, preferably linearly scaled slat for measurement of optimal diameter and depth of localization of the insert in the vagina, depending on actual and individual anatomical conditions of urogenital organs of the woman being treated.

The invention enables a method of treatment of prolapse of urogenital organs and urinary stress incontinence or in the period of intervals in women when the intravaginal therapeutic insert for treatment of static disorders of the urogenital organs and urinary stress incontinence is not currently inserted, by selecting the appropriate size of corrective insert from the sub-set of intravaginal corrective inserts, said selecting being realised by means of the intravaginal measuring sub-set, by the selection of appropriate optimal diameter and depth of localization of the corrective insert in vagina by approximations with using the balls from the measuring subset, so that contraction of the levator ani muscle will cause the elevation of the insert and the elevation of the insert will cause the elevation of the uterus and/or correction of the cysto-urethral angle, and during the progress of said treatment the sizes of successive applied corrective inserts are adjusted by analogic selection of appropriate optimal diameter and depth of localization of the measuring ball in the vagina, and advantageously by carrying out the exercises of the pelvic floor muscles of treated woman lying in prone genucubital position in the intervals between successive replacements of the corrective inserts.

An application of the corrective intravaginal insert enables maintaining of the insert in the vagina for an indefinite period of time. The insert diameter, selected by means of the measuring device, should ensure permanent correction of uterine and bladder placement, so that wall of the vagina was not too tense, and that a mass of lowered organs did not cause prolapse of the insert in the standing position.

Correction of the placement of the urogenital organs and reduction of the isometric contraction of muscles carrying urogenital organs result in restoration of normal blond supply, allowing them to regenerate and also prevent progressive lowering of the urogenital organs. Correction of the cysto-urethral angle enables control of the micturition.

The corrective insert from the set being the subject of the invention is used in the intervals between applications of the therapeutic insert and exerts beneficial effect on the muscles by removal of the isometric contraction and better preparation for dynamic contraction following application of the therapeutic insert. An alternating application of the therapeutic insert and the corrective insert enables the treatment of the more advanced cases of the urogenital prolapse and improves results of the therapy up to 80%.

The corrective insert applied alternately with the therapeutic insert, properly adjusted, supports the uterus in its optimal placement and, due to its spherical shape, it may move and turn freely in different directions, thus preventing. decubital ulceration.

The invention will now be described (by way of an illustrative example) in more detail with reference to the drawings, wherein Fig. 1 shows a sub-set of the corrective intravaginal inserts from the set being the subject of the invention, Fig. 2 shows the measuring sub-set from the set being the subject of the invention, Fig. 3 is a simplified sagittal cross-section of the female pelvis with the cysto-urethral angle enlarged almost up to 180°, when the micturition is started under control or in an involuntary way, Fig. 4 is a sagittal cross-section of the female pelvis with the corrective insert from the set being the subject of the invention causing correction of the cysto-urethral angle so that the resulting angle is right or obtuse; at this angle, the mechanism of urethral closure is most effective, and Fig, 5 is also a sagittal cross-section of the female pelvis and is showing a method of measuring the diameter of the vagina for determination of optimal localization and size of the corrective insert.

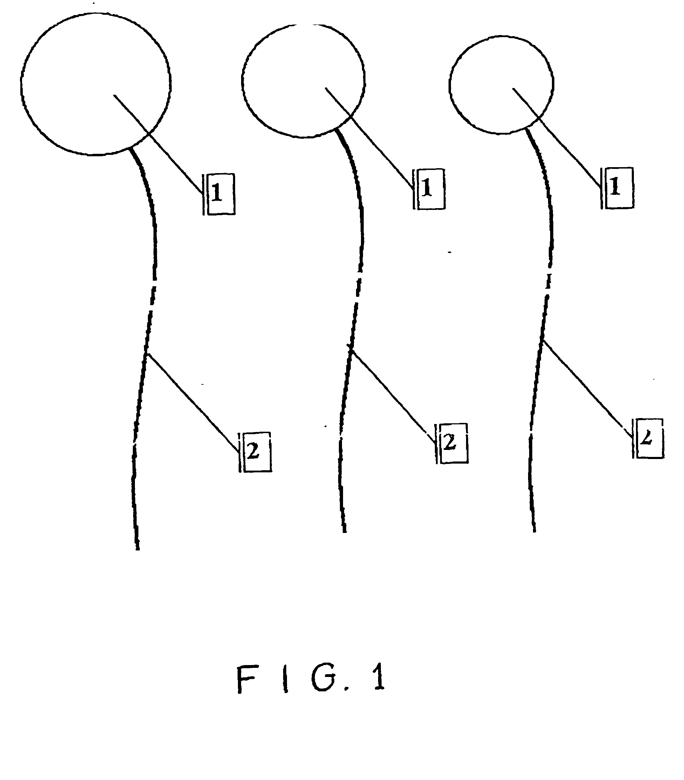

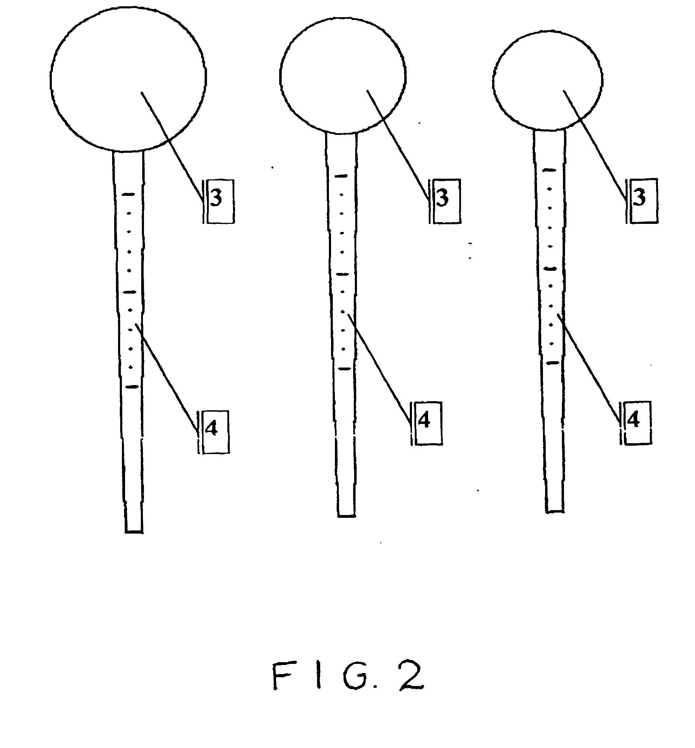

The intravaginal set being the subject of the invention consists of the sub-set of corrective intravaginal inserts (Fig. 1) being preferably the hollow balls 1 with step increase in diameter, ranging between the minimal and maximal vaginal diameter. Each ball 1 has the loosely hanging string 2 and is made preferably of medically inert plastics, such as polycarbonate or methyl methacrylate, and the set also consists of the intravaginal measuring sub-set (Fig. 2) containing measuring balls 3 made preferably of metal or plastic with graduated diameters corresponding to graduated diameters of balls 1 of the sub-set of corrective intravaginal inserts from Fig. 1; ,which balls 3 instead of the loosely hanging string 2 have, rigidly mounted, linearly scaled slat 4. The slat 4 is preferably scaled for measurement of diameter and optimal localization of the corrective insert in the vagina in the case of urinary stress incontinence

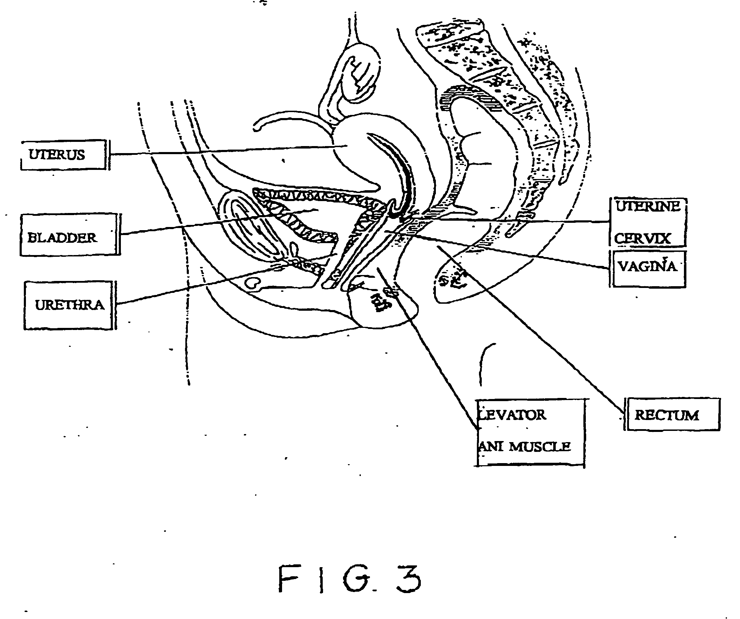

Fig. 3 shows a simplified sagittal cross-section of the female pelvis with the cysto-urethral angle enlarged almost up to 180°, when the micturition is controlled or involuntary.

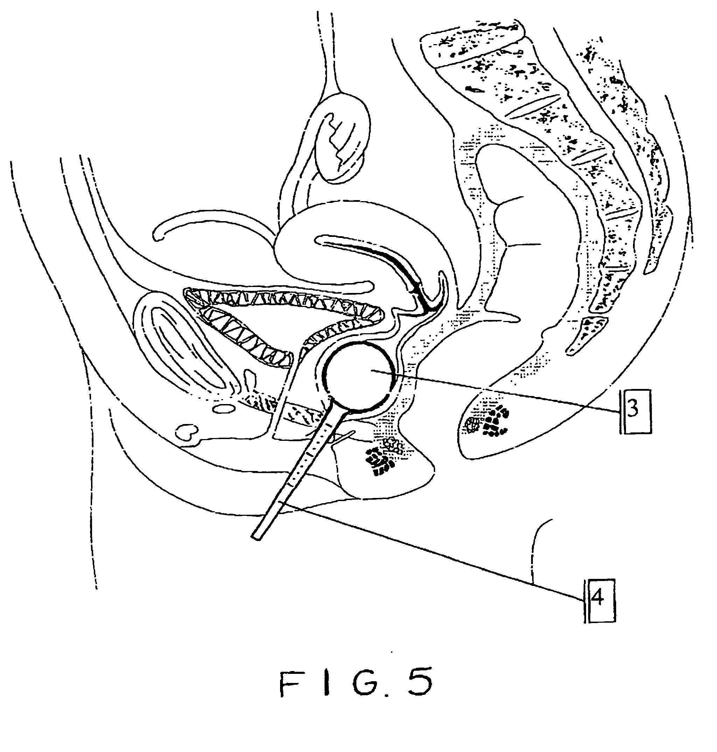

Fig. 5 shows the method of measuring diameter of the vagina using the ball 3 from the measuring sub-set for determination of depth of localization and size of the corrective insert. The appropriate anatomical size is adjusted using the balls 3 from the measuring sub-set. After the adjustment, it is checked, whether the measuring insert is not pushed out by the descending organs. In this case, a greater insert diameter should be applied. In the case of urgency, the insert is placed too high, then a smaller insert should be applied.

The application of the corrective intravaginal insert consists in determination of optimal diameter of the insert and intravaginal application of the corrective intravaginal insert for an indefinite period of time.

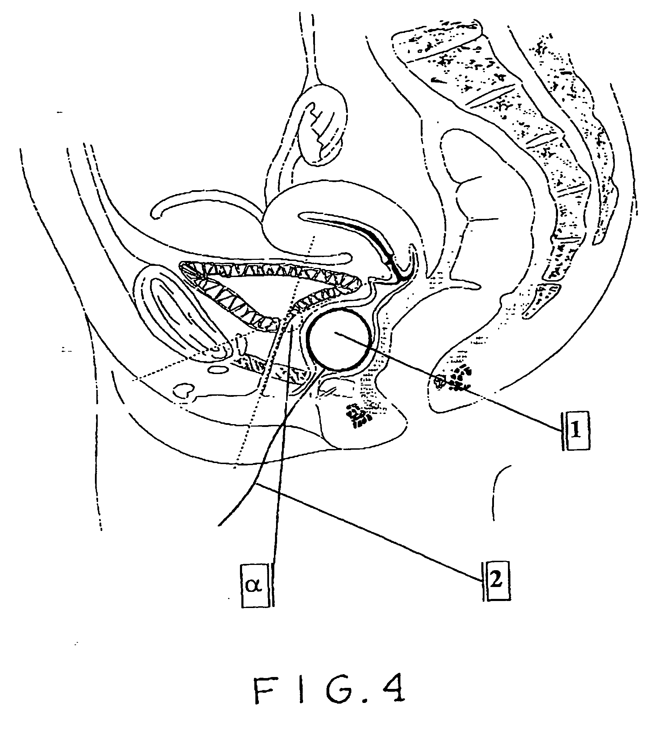

Fig. 4 shows a sagittal cross-section of the female pelvis with the corrective insert with properly selected diameter of the ball 1 by means of the measuring sub-set, and the string 2 is used for removal of the insert.

Mode of action of the insert is as follows: the insert being placed in the vagina is supported on the levator ani muscle. It results in forward and upward shift of the lowered anterior wall of the vagina and elevation of the urethra and urinary bladder. An elevation of the urethra restores its normal position in relation to the posterior wall of the urinary bladder (reduced cysto-urethral angle shown an Fig. 4). In the case of concomitant uterine prolapse, the insert causes elevation of the uterus.

An application of the corrective insert from the set being the subject of the invention results in very fast pain relief. The device also enables a controlled micturition due to correction of the cysto-urethral angle a without the necessity to remove the insert for micturition.

Displacement of the uterus to the "pure zone" enables complete removal of inflammation. Relaxation of muscles and fasciae restores normal blood supply of the organs of the pelvis minor. It is most probable that the corrective insert improves results of the treatment with the therapeutic insert. Alternating application of the therapeutic insert and the corrective insert enables the treatment of the more advanced cases of the urogenital descent and improves results of the therapy.

The insert can be easily applied and removed. It is supported an the posterior wall of the vagina at level of the levator muscle of anus, thus it prevents excessive extension of the vaginal walls.

The corrective insert from the set being the subject of the invention supports the prolapsed uterus and blocks the urethra in the case of urinary stress incontinence, inadequate urethral occlusion at increased intra-abdominal pressure. An optimal adjustment of insert diameter to the vagina allows the muscles to regenerate by reduction in uterine and cystic pressure. The corrective insert supports these organs, and after regeneration, the muscles will support the uterus and urinary bladder again, but in a corrected position.

A method of the treatment of prolapse of the urogenital organs and urinary stress incontinence in women is enabled with usage of the intravaginal set. What distinguishes this method is that the appropriate corrective insert is selected from the sub-set of corrective intravaginal inserts, consisting of at least two balls with step increase in diameter, ranging between the minimal and maximal vaginal diameter, each ball being preferably hollow, has the loosely hanging string and is made of medical material, such as polycarbonate or methyl methacrylate, and consisting of the intravaginal measuring sub-set that contains at least two metal or plastic balls with diameters corresponding to the diameters of the balls from corrective intravaginal sub-set which instead of the loosely hanging string, have, rigidly mounted, scaled slat for measurement of optimal diameter and localization of the insert in the vagina, depending on individual anatomical conditions of urogenital organ in woman, by selection of appropriate diameter and depth of vaginal localization by means of approximations with meaning balls from the measuring sub-set, so that contraction of the levator ani muscle causes elevation of the insert and elevation of the insert causes elevation of the uterus, and/or correction of the cysto-urethral angle, and during the progress of said treatment the sizes of successive applied inserts are adjusted by selection of appropriate diameter and depth of localization In the vagina and it is beneficial to carry out the exercises of the pelvic floor muscles laying on prone or genucubital position between the successive replacements of the insert.

Size of the applied insert gradually decreased under medical supervision using the measuring device and ensures permanent correction of uterine and bladder placement by correction of the cysto-urethral angle a, so that the muscle fibres diffused in surrounding tissue may restore the lowered organs to primary placement.

In the case of significant urogenital prolapse, the uterus is at the same level as the arms of the levator ani muscle, increasing distance between them.

In this case, an exercise of the levator ani muscle is disadvantageous, because muscular contraction exerts pressure on the descendent uterus without concomitant elevation. In this case the corrective intravaginal insert causes an elevation of the uterus, while contractions of the levator ani muscle elevates the insert and, indirectly, the uterus. The corrective insert may fall out at muscular contraction, when its diameter is too small. It is beneficial to make the test for contraction of the levator ani muscle following an adjustment of the corrective insert by the measuring device.

An exercise of the levator ani muscle with the usage of the corrective insert leads to improved efficiency of the levator ani muscle, vaginal stenosis and elevation of the uterus.

It is necessary to check position of the insert and the uterus within 2 weeks from the beginning of the recommended exercise.

In advanced cases of urogenital prolapse in which the uterine cervix is placed below the arms of the levator ani muscle, an exercise of the muscle with the usage of the corrective insert elevates the uterus with decrease in distance between both arms; the insert elevates, resulting in uterine elevation.

Progress in therapy may be monitored using the measuring device for examination of so-called pelvic floor thickness, i.e. following application, the measuring device is slightly pulled downward to rest it on the arms of the levator ani muscle and the distance from the pubic symphysis is read from the scaled slat.

An assessment of therapeutic effect includes repeated measurements of optimal diameter of the corrective insert.

## Claims

1. An intravaginal treatment set, useful in the case of prolapse of urogenital organs and urinary stress incontinence, or in the period of intervals in women when an intravaginal therapeutic insert for treatment of static disorders of the urogenital organs and urinary stress incontinence is not currently inserted, which therapeutic insert consists of hollow plastic ball with string attached and freely moving outside the ball, and with smaller ball, placed freely inside said hollow plastic ball, which smaller ball has a weight adequately adjusted to generate mechanical impulses stimulating alternate contractions of the smooth muscles, said treatment set comprising a sub-set of intravaginal corrective inserts for elevation of the uterus and a further sub-set of intravaginal measuring inserts for determining an optimal size for the corrective insert; wherein the sub-set of intravaginal corrective inserts comprises at least two corrective balls (1) with step increase in diameter, ranging between the minimal and maximal woman vaginal diameter, each said corrective ball having a loosely hanging string (2) for removing it from a vagina, while the intravaginal measuring sub-set comprises at least two balls (3) with graduated diameters corresponding to graduated diameters of the balls (1) of the sub-set of intravaginal corrective inserts, which measuring balls (3) instead of the removing means (2), have a slat (4) rigidly mounted on ech ball (3) of the measuring sub-set for measurement of a diameter and depth of localization of the corrective insert in the vagina, depending on actual and individual anatomical conditions of urogenital organs of the woman being treated, which is optimal for elevation of the uterus.

2. The intravaginal set as claimed in claim 1, **characterised in that**, each ball (1) of the sub-set of corrective inserts is hollow.

3. The intravaginal set as claimed in any one of the preceding claims, **characterised in that**, each ball (1) of the sub-set of corrective inserts is made from a medical material which is polycarbonate or methyl methacrylate.

4. The intravaginal set as claimed in any one of the preceding claims, **characterised in that**, each ball (3) of the measuring sub-set is made of metal or plastic.

5. The intravaginal set as claimed in claim 6, **characterised in that**, the slat (4) has a linear measuring scale.

## Patentansprüche

1. Intravaginales Behandlungsset, das bei einem Prolaps urogenitaler Organe und bei der Harnstressinkontinenz oder in den Zeitintervallen bei Frauen nützlich ist, in denen kein intravaginaler therapeutischer Einsatz zur Behandlung statischer Funktionsstörungen der urogenitalen Organe und der Harnstressinkontinenz gegenwärtig eingeführt ist, wobei der therapeutische Einsatz einen hohlförmigen Kunststoffball mit einem daran angebrachten Faden, der außerhalb des Balls frei beweglich ist, und einen kleineren Ball besitzt, der frei innerhalb des hohlförmigen Kunststoffballs angeordnet ist, wobei der kleinere Ball ein Gewicht besitzt, das geeignet ist, mechanische Impulse zu erzeugen, die abwechselnd Kontraktionen der weichen Muskeln stimulieren, und
wobei das Behandlungsset ein Unterset aus intravaginalen korrektiven Einsätzen zum Anheben des Uterus und ein weiteres Unterset aus intravaginalen Messeinsätzen zum Bestimmen einer optimalen Größe des korrektiven Einsatzes aufweist, bei dem das Unterset aus intravaginalen korrektiven Einsätzen mindestens zwei korrektive Bälle (1) mit stufenweiser Erhöhung des Durchmessers aufweist, der im Bereich zwischen dem minimalen und maximalen weiblichen Vaginaldurchmesser liegt, und wobei jeder korrektive Ball einen lose hängenden Faden (2) zum Entfernen desselben aus einer Vagina besitzt, und bei dem das intravaginale Mess-Unterset mindestens zwei Bälle (3) mit abgestuften Durchmessern entsprechend den abgestuften Durchmessern der Bälle (1) des Untersets aus intravaginalen korrektiven Einsätze aufweist, wobei die Messbälle (3) an Stelle des Entnahmemittels (2) einen Stab (4) besitzen, der fest an jedem Ball (3) des Mess-Untersets zum Messen eines Durchmessers und einer Ortstiefe des korrektiven Einsatzes in der Vagina in Abhängigkeit von tatsächlichen und individuellen anatomischen Bedingungen der urogenitalen Organe der in Behandlung befindlichen Frau besitzt, was zur Anhebung des Uterus optimal ist.

2. Intravaginales Set nach Anspruch 1, **dadurch gekennzeichnet, dass** jeder Ball (1) des Untersets aus korrektiven Einsätzen hohlförmig ist.

3. Intravaginales Set nach einem der vorhergehenden Ansprüche, **dadurch gekennzeichnet, dass** jeder Ball (1) des Untersets aus korrektiven Einsätzen aus einem medizinischen Material hergestellt ist, das Polycarbonat oder Methyl-Methacrylat entspricht.

4. Intravaginales Set nach einem der vorhergehenden Ansprüche, **dadurch gekennzeichnet, dass** jeder Ball (3) des Mess-Untersets aus Metall oder Kunststoff hergestellt ist.

5. Intravaginales Set nach Anspruch 6, **dadurch gekennzeichnet, dass** der Stab (4) eine lineare Messskala besitzt.

## Revendications

1. Ensemble de traitement intravaginal, utile dans le cas d'un prolapsus d'organes urogénitaux et d'incontinence d'urine à l'effort, ou lors de la période des intervalles chez la femme où un élément d'insertion thérapeutique intravaginal pour le traitement de troubles statiques d'organes urogénitaux et d'incontinence d'urine à l'effort n'est pas effectivement inséré, lequel élément d'insertion thérapeutique est constitué d'une bille en matière plastique creuse comportant un cordon fixé et se déplaçant librement à l'extérieur de la bille, et avec une bille plus petite, placée librement à l'intérieur de ladite bille en matière plastique creuse, laquelle bille plus petite présente un poids ajusté de façon adéquate pour générer des impulsions mécaniques stimulant des contractions alternées des muscles lisses, ledit ensemble de traitement comprenant un sous-ensemble d'éléments d'insertion correcteurs intravaginaux en vue d'une élévation de l'utérus et un autre sous-ensemble d'éléments d'insertion de mesure intravaginaux destiné à déterminer une taille optimale pour l'élément d'insertion correcteur, où le sous-ensemble d'éléments d'insertion correcteurs intravaginaux comprend au moins deux billes correctrices (1) présentant une augmentation de diamètre par étape, se situant entre le diamètre minimal et maximal du vagin de la femme, chaque dite bille correctrice comportant un cordon pendant librement (2) destiné à le retirer du vagin, tandis que le sous-ensemble de mesure intravaginal comprend au moins deux billes (3) ayant des diamètres progressifs correspondant aux diamètres progressifs des billes (1) du sous-ensemble d'éléments d'insertion correcteurs intravaginaux, lesquelles billes de mesure (3) à la place du moyen de retrait (2), comportent une lamelle (4) montée de façon rigide sur chaque bille (3) du sous-ensemble de mesure en vue de la mesure du diamètre et de la profondeur d'emplacement de l'élément d'insertion correcteur dans le vagin, en fonction des conditions anatomiques réelles et individuelles des organes urogénitaux de la femme qui est traitée, qui est optimal pour l'élévation de l'utérus.

2. Ensemble intravaginal selon la revendication 1, **caractérisé en ce que**, chaque bille (1) du sous-ensemble d'éléments d'insertion correcteurs est creuse.

3. Ensemble intravaginal selon l'une quelconque des revendications précédentes, **caractérisé en ce que**, chaque bille (1) du sous-ensemble d'éléments d'insertion correcteurs est réalisée à partir d'une matière médicale qui est un polycarbonate ou un méthacrylate de méthyle.

4. Ensemble intravaginal selon l'une quelconque des revendications précédentes, **caractérisé en ce que**, chaque bille (3) du sous-ensemble de mesure est constituée de métal ou de matière plastique.

5. Ensemble intravaginal selon la revendication 6, **caractérisé en ce que**, la lamelle (4) comporte une échelle de mesure linéaire.
